# EUROPEAN PATENT APPLICATION

(11) **EP 2 184 106 A1**
(43) Date of publication of application: **12.05.2010**
(21) Application number: 08777512.8
(22) Date of filing: 23.06.2008
(51) Int. Cl.: B01J 23/92, B01J 23/30, B01J 38/06, B01J 38/48, C07C 6/04, C07C 11/06, C07B 61/00

(54) **PROCESS FOR REACTIVATION OF METATHESIS CATALYSTS AND PROCESS FOR PRODUCTION OF OLEFINS COMPRISING THE REACTIVATION**

(30) Priority: 26.07.2007 JP 2007194918
(71) Applicant: Mitsui Chemicals, Inc., Tokyo 105-7117 (JP)
(72) Inventor: IKENAGA, Hirokazu, Sodegaura-shi Chiba 299-0265 (JP)
(74) Representative: Benson, John Everett
(86) International application number: PCT/JP2008/061399
(87) International publication number: WO 2009/013964

(57) **Abstract**

A metathesis catalyst which is a combination including a catalyst 1 comprising a compound that contains at least one metal element selected from tungsten, molybdenum and rhenium and a catalyst 2 comprising at least one selected from magnesium oxide and calcined hydrotalcite is easily and effectively reactivated from a degraded state due to long-term repetitive cycles of reaction and regeneration at high temperature for burning off poisonous substances or cokes, to like-new condition or a desired level. An olefin production process by a metathesis reaction includes a step of performing the reactivation.

A degraded metathesis catalyst is easily and effectively reactivated by being contacted with water at not more than 50°C or water vapor at not more than 170°C.

## Description

### FIELD OF THE INVENTION

The present invention relates to a method for easily and effectively reactivating a degraded metathesis catalyst that is a combination including a catalyst 1 comprising a compound that contains at least one metal element selected from tungsten, molybdenum and rhenium and a catalyst 2 comprising at least one selected from magnesium oxide and calcined hydrotalcite, by contacting the catalyst with water at not more than 50°C or water vapor at not more than 170°C. The invention also relates to an olefin production process by a metathesis reaction including a step of performing the reactivating method.

### BACKGROUND OF THE INVENTION

A metathesis reaction involves identical or different olefins that are reacted with each other and affords olefins having a differing structure. This reaction is very advantageous because it can cope with changes in olefin demands.

Olefin production processes by a metathesis reaction have been reported. For example, Patent Document 1 discloses a process of producing propylene by a metathesis reaction of ethylene and 2-butene using a catalyst mixture that contains a silica-supported tungsten oxide catalyst WO₃/SiO₂ and a magnesium oxide catalyst. Patent Document 2 discloses a process of producing propylene by a metathesis reaction of ethylene and n-butene wherein the catalytic activity is drastically improved by using a catalyst mixture containing WO₃/SiO₂ and magnesium oxide or calcined hydrotalcite in combination with a small amount of hydrogen.

However, the metathesis catalysts lower activity with time, though the deterioration degrees vary depending on reaction conditions, starting materials or catalyst types. For example, the catalytic activity is deteriorated over time due to catalyst poisoning by trace harmful substances contained in starting materials or due to coking that is deposition of heavy by-products on the catalysts. In a known catalyst regenerating method with these problems, an oxygen-containing gas is passed at a high temperature to burn off poisonous substances or cokes. Although this regeneration method is effective in the short term, repeated cycles of reaction and regeneration over a long period result in an activity that is not recovered to the desired level. It is therefore necessary that the catalysts are replaced regularly.

Patent Document 3 discloses a method for regenerating a magnesium oxide catalyst in which a Mn₂O₃-MgO catalyst that has been degraded in an alkylation reaction between a phenol and methanol is regenerated by contact with water at not more than 300°C. Because this alkylation reaction yields two equivalents of water, the Mn₂O₃ catalyst probably has high resistance to water. Further, because the reaction temperature is high ranging from 475 to 600°C, the water generated will be readily eliminated from the catalyst during the reaction. In contrast, tungsten, molybdenum and rhenium that are metathesis catalysts that are poisoned by water, and usually the metathesis reaction temperature is relatively low at not more than 350°C. Accordingly, there is concern that the catalysts may be adversely affected by contact with water.
Patent Document 1: US Patent No. 4,575,575
Patent Document 2: WO 2006/093058
Patent Document 3: JP-B-S59-006698

### SUMMARY OF THE INVENTION

### PROBLEMS TO BE SOLVED BY THE INVENTION

It is an object of the present invention that a metathesis catalyst which is a combination including a catalyst 1 comprising a compound that contains at least one metal element selected from tungsten, molybdenum and rhenium and a catalyst 2 comprising at least one selected from magnesium oxide and calcined hydrotalcite is easily and effectively reactivated from a degraded state due to long-term repetitive cycles of reaction and regeneration at high temperature for burning off poisonous substances or cokes, to like-new condition or a desired level. It is another object of the invention to provide an olefin production process by a metathesis reaction including a step of performing the reactivation. In the invention, the term "regeneration" refers to regeneration of metathesis catalysts by conventional techniques such as air calcination, and the term "reactivation" refers to regeneration by the method of the present invention.

The present inventors studied diligently to achieve the above objects. They have then found that a degraded metathesis catalyst which is a combination including a catalyst 1 comprising a compound that contains at least one metal element selected from tungsten, molybdenum and rhenium and a catalyst 2 selected from magnesium oxide and calcined hydrotalcite can be reactivated to like-new conditions for both the catalyst 1 and the catalyst 2 by contacting the metathesis catalyst with water at not more than 50°C or water vapor at not more than 170°C and sufficiently removing water by drying and calcination. The present invention has been completed based on the finding.

In a process of producing olefins by a metathesis reaction of starting olefins into different olefins, a catalyst reactivating method comprises bringing a degraded metathesis catalyst which is a combination including a catalyst 1 comprising a compound that contains at least one metal element selected from tungsten, molybdenum and rhenium and a catalyst 2 comprising at least one selected from magnesium oxide and calcined hydrotalcite into contact with water at not more than 50°C or water vapor at not more than 170°C. An olefin production process by a metathesis reaction according to the present invention comprises a step of performing the regenerating method.

In an aspect of the invention, there is provided a process for producing propylene by a metathesis reaction between ethylene and n-butene in the presence of the metathesis catalyst comprising the catalyst 1 and the catalyst 2 that are reactivated by the above method.

### ADVANTAGEOUS EFFECTS OF THE INVENTION

According to the methods of the present invention, the metathesis catalyst performance is regenerated effectively, although the compound used is only water without any special equipment for metathesis catalyst reactivation, and olefins can be produced with significant advantages in the aspects of safety, processing and economics.

### BRIEF DESCRIPTION OF THE DRAWING

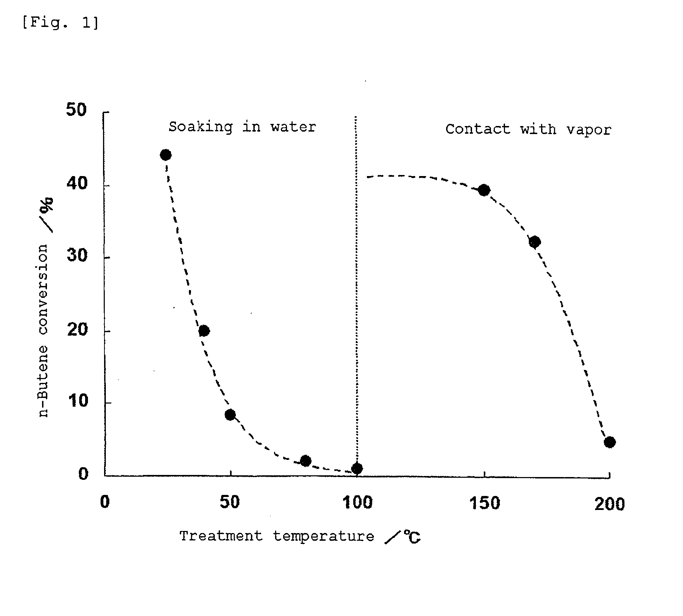

Fig. 1 is a graph showing relations of the water treatment temperature and the butene conversion in Examples of the invention.

### PREFERRED EMBODIMENTS FOR CARRYING OUT THE INVENTION

The catalyst 1 used in the invention contains at least one metal element selected from tungsten, molybdenum and rhenium. The structures of tungsten, molybdenum and rhenium are not limited, and oxides, sulfides and hydroxides may be used. In particular, oxides such as WO₃, MoO₃ and Re₂O₇ are preferable, and WO₃ is more preferable. These oxides, sulfides or hydroxides may be supported on inorganic compounds called supports. The kinds of supports are not particularly limited, and examples thereof include silica, alumina and titania, with silica being particularly preferable. The supporting methods used may be conventional for the skilled in the art and are not particularly limited. The amount of the metal element relative to the support may be in the range of 0.01 wt% to 50 wt%, and more preferably 0.1 wt% to 20 wt% in terms of oxide.

The catalyst 2 used in the invention includes at least one selected from magnesium oxide and calcined hydrotalcite. In the invention, the calcined hydrotalcite is a MgO·A1₂O₃ solid solution obtained by calcining at 300°C or above a hydrotalcite of Formula 1 below which is a layered magnesium-aluminum double hydroxide.

[Chem. 1] [Mg²⁺₁₋ₓAl³⁺ₓ(OH)₂]^{x+}[(Aⁿ⁻)x/n·mH₂O]^{x-} 1

The letter A is an anion, n is a valence of the anion A, x usually ranges from 0.20 to 0.33, and m is usually an integer of about 0 to 4 although greatly varied depending on the dehydration degree.

Examples of the anions include carbonate ion, sulfate ion, hydroxide ion, fluoride ion, chloride ion, bromide ion and iodide ion.

The magnesium oxide or hydrotalcite may be used as it is, or these oxides may be supported on inorganic compounds called supports. These compounds may be obtained by known methods in the art without limitation. For example, hydroxides, carbonates or the like may be decomposed at high temperatures. The supporting methods may be conventional in the art without limitation. The supports are not particularly limited, and examples thereof include silica, alumina and titania, with silica being preferable.

The shapes of the catalysts 1 and 2 are not particularly limited. The sizes thereof may be selected appropriately depending on the size of reactors. The catalysts may be shaped by known methods in the art without limitation.

The catalyst 1 and the catalyst 2 may be physically mixed together or may be supported together on a single support. The metathesis catalyst may contain the catalyst 1 and the catalyst 2 at any proportions without limitation but will catalyze a metathesis reaction more effectively when the weight ratio of the catalyst 2 is 50% or more relative to the total catalyst weight.

Examples of the starting olefins in the metathesis reaction include linear or cyclic olefins of 2 to 10 carbon atoms. Starting olefins including ethylene and butene give propylene. Starting olefins including butene and pentene give hexene. The starting olefins may contain paraffins such as methane, ethane, propane, butane and hexane, and hydrogen. The presence of hydrogen drastically accelerates the metathesis reaction.

The temperature and pressure in the metathesis reaction are generally in the range of 25 to 500°C and 0.1 to 20 MPaG, and preferably 100 to 400°C and 0.1 to 10 MPaG. The amount of the catalysts is not particularly limited. For example, when the reaction is carried out using a fixed-bed flow apparatus, WHSV is preferably in the range of 1 to 500 h⁻¹, and more preferably 1 to 250 h⁻¹ wherein the WHSV represents the amount (weight) of starting materials per unit time divided by the weight of the catalyst.

The metathesis reaction may be carried out in any mode without limitation, but a gas phase flow reaction is preferable. The catalyst packing modes include fixed beds, fluidized beds and suspended beds. Any method may be applicable.

The reactivation step of the invention is usually carried out before or after the usual regeneration step, and is more preferably performed after the usual regeneration step.

In the usual regeneration treatment in a metathesis reaction, an oxygen-containing gas is passed at a high temperature to burn off poisonous substances or cokes from the catalyst. Known regeneration methods in the art may be used without limitation.

After the completion of the reactivation step, it is desired that the metathesis reaction is initiated after a reduction treatment with a reductive gas such as carbon monoxide or hydrogen as is generally conducted in the art. Known reduction methods in the art may be used without limitation.

The water at not more than 50°C used in the process of the invention is liquid phase water. The catalysts 1 and 2 may be soaked in the water, or the water may be passed through the catalysts 1 and 2. The water temperature is preferably 0 to 50°C, and more preferably 20 to 40°C. If the water temperature exceeds 50°C, the catalysts cannot be reactivated sufficiently. The pressure is not particularly limited, but is preferably normal pressure at room temperature for simple processing.

The time of contact with water at not more than 50°C is not particularly limited, but is preferably in the range of 30 minutes to 1 week, and more preferably 1 hour to 24 hours. The contact is preferably followed by drying and calcination to remove water sufficiently. The drying is usually performed at 100 to 300°C for 1 to 120 hours, and particularly preferably at 100 to 200°C for 1 to 24 hours. The calcination is usually performed at 350 to 700°C for 1 to 120 hours, and particularly preferably at 400 to 600°C for 1 to 24 hours. The drying and calcination may be conducted in any atmosphere without limitation, and may be carried out for example under vacuum (evacuation), under a stream of inert gas, or under a stream of air or hydrogen gas.

The water vapor at not more than 170°C used in the invention is gas phase water that is brought into contact with the catalysts 1 and 2. For example, the catalysts 1 and 2 may be allowed to stand in the water vapor, or the water vapor may be continuously passed through the catalysts 1 and 2. The water vapor temperature is preferably 100 to 170°C, and more preferably 110 to 160°C. If the water vapor temperature exceeds 170°C, the catalysts cannot be regenerated sufficiently. The pressure is not particularly limited, but the catalysts are reactivated more quickly as the pressure is closer to the saturated vapor pressure of water at the processing temperature.

The time of contact with water vapor at not more than 170°C is not particularly limited, but is preferably in the range of 30 minutes to 1 week, and more preferably 1 hour to 24 hours. The contact is preferably followed by drying and calcination to remove water sufficiently. The drying is usually performed at 100 to 300°C for 1 to 120 hours, and particularly preferably at 100 to 200°C for 1 to 24 hours. The calcination is usually performed at 350 to 700°C for 1 to 120 hours, and particularly preferably at 400 to 600°C for 1 to 24 hours. The drying and calcination may be conducted in any atmosphere without limitation, and may be carried out for example under vacuum (evacuation), under a stream of inert gas, or under a stream of air or hydrogen gas. The contacting methods, drying methods and calcination methods described above do not restrict the scope of the invention.

The methods of the invention are advantageous in that the catalyst can be reactivated in the metathesis reactor. In detail, the catalyst may be taken out from the reactor and be reactivated in a separate reactivation container; alternatively, a pipe or the like for supplying water or water vapor may be attached to the reactor and the catalyst may be reactivated in the packed state without being collected from the reactor. In terms of switching between the reaction and the catalyst reactivation, the catalyst is preferably reactivated in the reactor as in the latter case.

### [EXAMPLES]

Hereinbelow, the present invention will be described in greater detail based on examples without limiting the scope of the invention.

### (Example 1)

A metathesis reaction of ethylene and 2-butene into propylene was studied. A reactor was packed with a catalyst prepared by physically mixing 12 g of WO₃/SiO₂ in which WO₃ was supported on SiO₂ and 48 g of magnesium oxide. The catalyst was pretreated by air calcination at 550°C followed by hydrogen reduction at 550°C. The temperature was then lowered to 350°C, which was predetermined as the reaction temperature. Subsequently, ethylene and 2-butene were supplied to the reactor in amounts such that the molar ratio of ethylene to 2-butene would be 1.5 and the weight hourly space velocity (WHSV) would be 30 h⁻¹. The weight hourly space velocity was a ratio of the total flow rate of ethylene and 2-butene relative to the amount of WO₃/SiO₂. The reaction pressure was 0 MPaG. The conversion was calculated from the proportion of n-butene consumed in the reaction. The main product was propylene. The conversion after 10 hours of the reaction initiation was 43.5%.

After 20 hours of the reaction, the supply of the starting materials was suspended. The catalyst was regenerated by air calcination at 550°C for 10 hours. After the regeneration by air calcination, the reaction was reinitiated. The conversion after 10 hours of the reaction was 43.0%, showing that the activity was slightly lowered after one regeneration treatment. This cycle of metathesis reaction and regeneration was repeated 20 times at regular intervals, and the catalyst that had undergone 20th regeneration by air calcination was used to catalyze the reaction. The conversion after 10 hours of the reaction was only 0.5%. A degraded catalyst was thus obtained.

Subsequently, 0.6 g of the degraded catalyst was soaked and was left still in approximately 50 ml of distilled water at 25°C for 24 hours. The catalyst was then dried at 130°C for 8 hours and was air calcined at 500°C for 2 hours, resulting in a reactivated catalyst. The reactivated catalyst was used to catalyze the metathesis reaction. After 10 hours of the reaction, the conversion was 44.5%, nearly the same level as the fresh catalyst. The results are set forth in Fig. 1.

### (Example 2)

The procedures of Example 1 were repeated, except that the catalyst was soaked in water at 40°C. The reactivated catalyst was used to catalyze the metathesis reaction. After 10 hours of the reaction, the conversion was 20.0%. The results are set forth in Fig. 1.

### (Example 3)

The procedures of Example 1 were repeated, except that the catalyst was soaked in water at 50°C. The reactivated catalyst was used to catalyze the metathesis reaction. After 10 hours of the reaction, the conversion was 8.5%. The results are set forth in Fig. 1.

### (Example 4)

A degraded catalyst was prepared as described in Example 1. Water vapor was passed through 0.6 g of the degraded catalyst at 150°C and 0.2 MPaG at a rate of 0.0125 g/min for 24 hours. The gas supply was switched from water vapor to air, and the catalyst was dried at 150°C for 2 hours and was calcined at 500°C for 2 hours, resulting in a reactivated catalyst. The reactivated catalyst was used to catalyze the metathesis reaction. After 10 hours of the reaction, the conversion was 39.3%, nearly the same level as the fresh catalyst. The results are set forth in Fig. 1.

### (Example 5)

The procedures of Example 4 were repeated, except that the catalyst was treated with water vapor at 170 °C and 0.6 MPaG. The reactivated catalyst was used to catalyze the metathesis reaction. After 10 hours of the reaction, the conversion was 32.4%. The results are set forth in Fig. 1.

### (Example 6)

A metathesis reaction of ethylene and 2-butene into propylene was studied. A reactor was packed with a catalyst prepared by physically mixing 12 g of WO₃/SiO₂ in which WO₃ was supported on SiO₂ and 48 g of calcined hydrotalcite. The catalyst was pretreated by air calcination at 550°C followed by hydrogen reduction at 550°C. The temperature was then lowered to 350°C, which was predetermined as the reaction temperature. Subsequently, ethylene and 2-butene were supplied to the reactor in amounts such that the molar ratio of ethylene to 2-butene would be 1.5 and the weight hourly space velocity (WHSV) would be 30 h⁻¹. The weight hourly space velocity was a ratio of the total flow rate of ethylene and 2-butene relative to the amount of WO₃/SiO₂. The reaction pressure was 0 MPaG. The conversion was calculated from the proportion of n-butene consumed in the reaction. The main product was propylene. The conversion after 17 hours of the reaction initiation was 64.4%.

After 20 hours of the reaction, the supply of the starting materials was suspended. The catalyst was regenerated by air calcination at 550°C for 10 hours. After the regeneration, the reaction was reinitiated. The conversion after 10 hours of the reaction was 64.0%, showing that the activity was slightly lowered after one regeneration treatment. This cycle of metathesis reaction and regeneration was repeated 20 times at regular intervals, and the catalyst that had undergone 20th regeneration was used to catalyze the reaction. The conversion after 10 hours of the reaction was only 50.8%. A degraded catalyst was thus obtained.

Subsequently, 0.6 g of the degraded catalyst was soaked and was left still in approximately 50 ml of distilled water at 25°C for 24 hours. The catalyst was then dried at 130°C for 8 hours and was air calcined at 500°C for 2 hours, resulting in a reactivated catalyst. The reactivated catalyst was used to catalyze the metathesis reaction. After 10 hours of reaction, the conversion was 62.9%, nearly the same level as the fresh catalyst.

### (Comparative Example 1)

The procedures of Example 1 were repeated, except that the catalyst was soaked in water at 80°C or 100°C. The reactivated catalyst was used to catalyze the metathesis reaction. After 10 hours of the reaction, the conversion by the catalyst reactivated at 80°C was 2.0%, and that by the catalyst reactivated at 100°C was 1.2%. The results are set forth in Fig. 1.

### (Comparative Example 2)

The procedures of Example 4 were repeated, except that the catalyst was treated with water vapor at 200°C and 0.2 MPaG. The reactivated catalyst was used to catalyze the metathesis reaction. After 10 hours of the reaction, the conversion was 5.0%. The results are set forth in Fig. 1.

## Claims

1. In a process of producing olefins by a metathesis reaction of starting olefins into different olefins, a catalyst reactivating method comprising bringing a degraded metathesis catalyst which is a combination including a catalyst 1 comprising a compound that contains at least one metal element selected from tungsten, molybdenum and rhenium and a catalyst 2 comprising at least one selected from magnesium oxide and calcined hydrotalcite into contact with water at not more than 50°C or water vapor at not more than 170°C.

2. An olefin production process by a metathesis reaction, comprising a step of performing the reactivating method of claim 1 in which the metathesis catalyst including the catalyst 1 and the catalyst 2 is reactivated.

3. A metathesis catalyst comprising the catalyst 1 and the catalyst 2 that are reactivated by the method of claim 1.

4. A process for producing propylene by a metathesis reaction between ethylene and n-butene in the presence of the metathesis catalyst comprising the catalyst 1 and the catalyst 2 that are reactivated by the method of claim 1.
